# EUROPÄISCHE PATENTSCHRIFT

(11) **EP 0 007 066 B1**
(45) Veröffentlichungstag und Bekanntmachung des Hinweises auf die Patenterteilung: **03.06.1981**
(21) Anmeldenummer: 79102271.8
(22) Anmeldetag: 04.07.1979
(51) Int. Cl.: C07D 239/47, A01N 43/54

(54) **4-Alkyl- und 4-Allyl-merkapto-, sulfinyl- und sulfonyl-methyl-2-amino-6-N,N'-dimethylcarbamoyloxy-pyrimidine, Verfahren zu ihrer Herstellung, Mittel welche diese Pyrimidine enthalten und deren Verwendung zur Bekämpfung von Insekten**
4-Alkyl- and 4-allyl-thio-, sulfinyl- and sulfonyl-methyl-2-amino-6-N,N'-dimethylcarbamoyloxy pyrimidines, process for their preparation, compositions containing them and their use as insecticides
4-Alkyl- et 4-allyl-thio-, sulfinyl- et sulfonyl-méthyl-2-amino-6-N,N'-diméthylcarbamoyloxy-pyrimidines, procédé pour leur préparation, compositions les contenant et leur utilisation comme insecticides

(30) Priorität: 07.07.1978 CH 7424/78; 07.06.1979 CH 5308/79
(43) Veröffentlichungstag der Anmeldung: 23.01.1980
(73) Patentinhaber: CIBA-GEIGY AG, 4002 Basel (CH)
(72) Erfinder: Drabek, Jozef, Dr., CH-4104 Oberwil (CH)
(74) Vertreter: Zumstein sen., Fritz

## Beschreibung

Die vorliegende Erfindung betrifft neue 4-Alkyl- und 4-Allyl-merkapto-, sulfinyl- und sulfonylmethyl-2-amino-6-N,N-dimethylcarbamoyloxy-pyrimidine, welche eine Wirkung gegen Insekten besitzen, und Verfahren zu ihrer Herstellung sowie insektizide Mittel, welche die vorstehend genannten Pyrimidine als aktive Komponente enthalten, und Verfahren zur Bekämpfung von Insekten unter Verwendung der neuen Verbindungen.

2-Aminopyrimidin-6-carbamate mit pestizider Wirkung sind bekannt (vgl. GB-Patentschrift Nr. 1,181,657). Nach vorliegender Erfindung werden neuartige Verbindungen dieses Typs bereitgestellt, welche sich durch eine besonders stark ausgeprägte, gegen Insekten der Ordnung Homoptera vor allem der Familie Aphididae spezifische Wirkung auszeichnen, und welche aufgrund ihrer vorteilhaften biologischen Eigenschaften für die praktische Anwendung auf dem Gebiet der Schädlingsbekämpfung besonders geeignet sind.

Die erfindungsgemässen 4-Alkyl- und 4-Allyl-merkapto-, sulfinyl- und sulfonyl-methyl-2-amino-6-N,N-dimethylcarbamoyloxy-pyrimidine entsprechen der Formel I 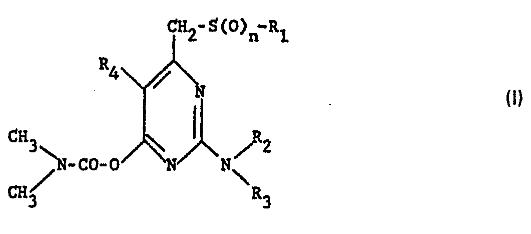 worin
R₁ eine C,-c₄₋Alkyl- oder Allylgruppe,
R₂ ein Wasserstoffatom oder eine Methyl- oder Aethylgruppe,
R₃ eine Methyl-, Aethyl- oder Cyclopropylgruppe,
R₄ ein Wasserstoffatom oder eine Methylgruppe und n die Zahl 0, 1 oder 2 bedeuten.

Die für R, in Frage kommenden Alkylgruppen sind die Methyl-, Aethyl-, n-Propyl- und i-Propylgruppe sowie die n-, i-, sek.- und tert.-Butylgruppe.

In den Verbindungen der Formel I werden die folgenden Substituententypen sowie Kombinationen derselben untereinander bevorzugt:
1) Bei R₁: C₁―C₃-Alkyl insbesondere Methyl and Aethyl;
2) Bei R₂: Wasserstoff oder Methyl, bei R₃: Methyl und bei R₄: Wasserstoff;
3) Bei n: die Zahl O.

Die neuen Verbindungen der Formel I werden zweckmässig nach an sich bekannten Methoden dadurch erhalten, dass man z.B.
a) eine Verbindung der Formel II 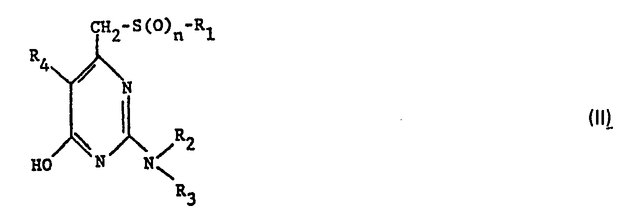 in Gegenwart einer Base mit einem Dimethylcarbamoylhalid insbesondere mit Dimethylcarbamoylchlorid umsetzt; oder dass man
b) eine Verbindung der Formel III 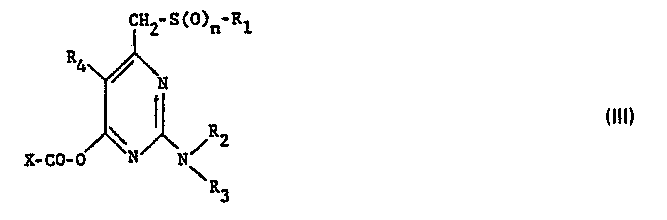 gegebenenfalls in Gegenwart einer geeigneten Base mit Dimethylamin reagieren lässt; wobei in den Formeln 11 und III R,, R₂, R₃, R₄ und n die unter Formel bereits angegebenen Bedeutungen haben und X für ein Halogenatom insbesondere ein Chloratom steht.

Das Verfahren (a) wird zweckmässig bei einer Temperatur zwischen 20° und 130°C, vorzugsweise zwischen 50° und 100°C und das Verfahren (b) bei einer Temperatur zwischen 50° und: 100°C vorgenommen.

Beide Reaktionen werden bei normalen oder leicht erhöhtem Druck gegebenenfalls in Gegenwart eines gegenüber den Reaktionsteilnehmern inerten Lösungs- oder Verdünnungsmittel durchgeführt. Als Lösungs- oder Verdünnungsmittel eignen sich z.B. Aether und ätherartige Verbindungen wie Diäthyläther, Di-isopropyläther, Dioxan und Tetrahydrofuran; aromatische Kohlenwasserstoffe wie Benzol, Toluol und Xylole; Ketone wie Aceton, Methyläthylketon und Cyclohexanon; Nitrile wie Acetonitril; sowie Dimethylformamid und Hexamethylphosphorsäuretriamid.

Als für diese Verfahren geeignete Basen kommen insbesondere tertiäre Amine wie Trialkylamine und Dialkylaniline ferner Hydroxide, Oxide, Carbonate und Bicarbonate von Alkali- und Erdalkalimetallen sowie Alkalimetallalkoholate wie Kallium-tert.butylate und Natrium-methylat in Betracht.

Die Ausgangsstoffe der Formel II und III sind aus bekannten Vorläufern erhältlich, z.B. nach dem folgenden Reaktionsschema:

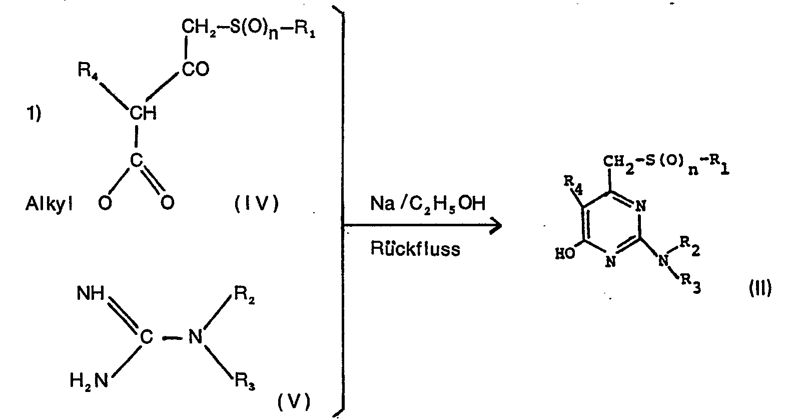

(Verbindungen der Formel II, worin n die Zahl 1 oder 2 bedeutet, können auch durch Umsetzung der entsprechenden Verbindung der Formel II, worin n die Zahl 0 ist mit einem Oxydationsmittel, wie z.B. H₂0₂ oder einer organischen Peroxid-Verbindung oder Persäure, hergestellt werden.)

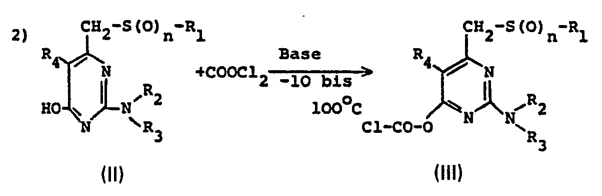

Es hat sich nun überraschenderweise gezeigt, dass die Verbindungen der Formel 1 eine ausgezeichnete Wirkung gegen Insekten, in erster Linie gegen pflanzenschädigende Insekten der Familie Aphididae, aufweisen. Dabei wurde festgestellt, dass die erfindungsgemässen Verbindungen sowohl eine Kontakt- als auch systemische Wirkung gegen verschiedenste Vertreter der genannten Familie (wie z.B. Aphis fabae, Aphis craccivora und Myzus persicae), welche sich mit bekannten Mitteln nur schwierig bekämpfen lassen, besitzen. Darüber hinaus wurde weiter festgestellt, dass die erwähnten aphiziden Eigenschaften mit einer bei der Verwendung auf dem Gebiete der Landwirtschaft vorteilhaft niedrigen Toxizität gegenüber Warmblütern gekoppelt sind. Diesen Eigenschaften zufolge, sind die Verbindungen der Formel 1 erfindungsgemäss zur Bekämpfung von Insekten, vor allem von Aphiden, in Kulturen von Nutz- und Zierpflanzen, hauptsächlich in Obst- und Gemüsekulturen, besonders geeignet.

Die insektizide Wirkung der erfindungsgemässen Verbindungen lässt sich durch Zusatz von anderen Insektiziden und/oder Akariziden wesentlich verbreitern und an gegebene Umstände anpassen.

Als Zusätze eignen sich z.B.: org.Phosphorverbindungen; Nitrophenole und deren Derivate; Formamidine; Harnstoffe; pyrethrinartige Verbindungen; Karbamate und chlorierte Kohlenwasserstoffe.

Die Verbindungen der Formel I können für sich allein oder zusammen mit geeigneten Trägern und/oder Zuschlagstoffen eingesetzt werden. Geeignete Träger und Zuschlagstoffe können fest oder flüssig sein und entsprechen den in der Formulierungstechnik üblichen Stoffen wie z.B. natürlichen oder regenerierten Stoffen, Lösungs-, Dispergier-, Netz-, Haft-, Verdickungs-, Binde- und/oder Düngemitteln.

Zur Applikation können die Verbindungen der Formel I zu Stäubemitteln, Emulsionskonzentraten, Granulaten, Dispersionen, Sprays, zu Lösungen oder Aufschlämmungen in üblicher Formulierung, die in der Applikationstechnik zum Allgemeinwissen gehören, verarbeitet werden.

Die Herstellung erfindungsgemässer Mittel erfolgt in an sich bekannter Weise durch inniges Vermischen und/oder Vermahlen von Verbindungen der Formel I mit den geeigneten Trägerstoffen, gegebenenfalls unter Zusatz von gegenüber den Wirkstoffen inerten Dispergier- oder Lösungsmitteln. Die Wirkstoffe (Verbindungen der Formel 1) können in den folgenden Aufarbeitungsformen vorliegen und angewendet werden:
Feste
Aufarbeitungsformen: Stäubemittel, Streumittel und Granulate (Umhüllungsgranulate, Imprägnierungsgranulate und Homogengranulate);
Flüssige

Aufarbeitungsformen:
a) in Wasser dispergierbare Spritzpulver, Pasten und Emulsionen;
b) Lösungen.

Der Gehalt an Wirkstoff (Verbindung der Formel I) in den oben beschriebenen Mitteln liegt zwischen 0,1 bis 95%, dabei ist zu erwähnen, dass bei der Applikation aus dem Flugzeug oder mittels anderer geeigneter Applikationsgeräte auch höhere Konzentrationen eingesetzt werden können.

Die Wirkstoffe der Formel I können beispielsweise wie folgt formuliert werden:
Stäubemittel: Zur Herstellung eines a) 5%igen und b) 2%igen Stäubemittels werden die folgenden Stoffe verwendet:
   a) 5 Teile Wirkstoff, 95 Teile Talkum;
   b) 2 Teile Wirkstoff, 1 Teil hochdisperse Kieselsäure, 97 Teile Talkum.

Die Wirkstoffe werden mit den Trägerstoffen vermischt und vermahlen.

Granulat: Zur Herstellung eines 5%igen Granulates werden die folgenden Stoffe verwendet:
5 Teile Wirkstoff,
0,25 Teile Epichlorhydrin,
0,25 Teile Cetylpolyglykoläther,
3,50 Teile Polyäthylenglykol,
91 Teile Kaolin (Korngrösse 0,3 -0,8 mm).

Der Wirkstoff wird mit Epichlorhydrin vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton im Vakuum verdampft.

Spritzpulver: Zur Herstellung eines a) 40%igen, b) und c) 25%igen d) 10%igen Spritzpulvers werden folgende Bestandteile verwendet:
a) 40 Teile Wirkstoff, 5 Teile Ligninsulfonsäure-Natriumsalz, 1 Teil Dibutylnaphthalinsulfonsäure-Natriumsalz, 54 Teile Kieselsäure;
b) 25 Teile Wirkstoff, 4,5 Teile Calcium-Ligninsulfonat, 1,9 Teile Champagne-Kreide/Hydroxyäthylcellulose-Gemisch (1:1), 1,5 Teile Natrium-dibutyl-naphthalinsulfonat, 19,5 Teile Kieselsäure.. 19,5 Teile Champagne-Kreide, 28,1 Teile Kaolin;
c) 25 Teile Wirkstoff, 2,5 Teile Isooctylphenoxy-polyäthylen-äthanol, 1,7 Teile Champagne-Kreide/Hydroxyäthylcellulose-Gemisch (1:1), 8,3 Teile Natriumaluminiumsilikat, 16,5 Teile Kieselgur, 46 Teile Kaolin;
d) 10 Teile Wirkstoff, 3 Teile Gemisch der Natriumsalze von gesättigten Fettalkoholsulfaten, 5 Teile Naphthalinsulfonsäure/Formaldehyd-Kondensat, 82 Teile Kaolin;

Die Wirkstoffe werden in geeigneten Mischern mit den Zuschlagstoffen innig vermischt und auf entsprechenden Mühlen und Walzen vermahlen. Man erhält Spritzpulver, die sich mit Wasser zu Suspensionen der gewünschten Konzentration verdünnen lassen.

Emulgierbare Konzentrate: Zur Herstellung eines a) 10%igen b) 25%igen und c) 50%igen emulgierbaren Konzentrates werden folgende Stoffe verwendet:
a) 10 Teile Wirkstoff, 3,4 Teile epoxyidertes Pflanzenöl, 3,4Teile eines Kombinationsemulgators, bestehend aus Fettalkoholpolyglykoläther und Alkylarylsulfonat-Calcium-Salz, 40 Teile Dimethylformamid und 43,2 Teile Xylol;
b) 25 Teile Wirkstoff, 2,5 Teile epoxydiertes Pflanzenöl, 10 Teile eines Alkylarylsulfonat/Fettalkoholpolyglykoläther-Gemisches, 5 Teile Dimethylformamid und 57,5 Teile Xylol;
c) 50 Teile Wirkstoff, 4,2 Teile Tributylphenol-Polyglykoläther, 5,8 Teile Ca-Dodecylbenzolsulfonat, 20 Teile Cyclohexanon, 20 Teile Xylol.

Aus solchen Konzentraten können durch Verdünnen mit Wasser Emulsionen der gewünschten Konzentration hergestellt werden. Sprühmittel. Zur Herstellung eines a) 5%igen und b) 95%igen Sprühmittels werden die folgenden Bestandteile verwendet:
a) 5 Teile Wirkstoff, 1 Teil Epichlorhydrin, 94 Teile Benzin (Siedegrenzen 160-190°C)
b) 95 Teile Wirkstoff, 5 Teile Epichlorhydriri.

Die nachfolgenden Beispiele dienen zur näheren Erläuterung der Erfindung:
Beispiel 1: Herstellung von 2-Dimethylamino-4.-äthylmerkaptomethyl-6-N,N-dimethylcarbamoyloxy- pyrimidin.

### a) Herstellung des Ausgangsstoffes: 2-Dimethylamino-4-äthylmerkaptomethyl-6-hydroxy-pyrimidin.

Zu einer Lösung von 8,1 g Natrium in 300 ml abs. Aethanol wurde bei Raumtemperatur portionsweise 47,9 g Dimethylguanidinsulfat zugegeben. Das erhaltene Gemisch wurde während einer Stunde bei Raumtemperatur und anschliessend noch weitere 30 Minuten am Rückfluss gerührt. Nach der Abkühlung wurde 62,0 g γ-Aethylmerkapto-acetessigsäure-methyl-ester zugetropft und die erhaltene Reaktionsmischung während 24 Stunden am Rückfluss gekocht. Nach der Abkühlung wurde das erhaltene Reaktionsgemisch auf Eis/Wasser gegossen und das pH auf 7 gestellt. Nach dem Extrahieren des Reaktionsgemisches mit Methylenchlorid wurde die organische Phase über Na₂S0₄ getrocknet, das Lösungsmittel abdestilliert und das erhalten Rohprodukt aus Wasser umkristallisiert. Auf diese Weise erhielt man das 2-Dimethylamino-4-äthylmerkaptomethyl-6-hydroxy-pyrimidin als farblose Kristalle mit einem Smp. von 119-121°C.

### b) Herstellung des 2-Dimethylamino-4-äthylmerkaptomethyl-6-N,N-dimethylcarbamoyloxy-pyrimidins.

Zu einer Lösung von 14,9 g 2-Dimethylamino-4-äthylmerkaptomethyl-6-hydroxy-pyrimidin in 100 ml Methyläthylketon wurde 9,7 g K₂C0₃ und 0,4 ml Triäthylamin zugegeben. Nach dem Rühren des Gemisches während 2 Stunden am Rückfluss wurde unter Rühren 7,5 g Dimethylcarbamoylchlorid zugetropft und das erhaltene Reaktionsgemisch anschliessend während 8 Stunden am Rückfluss gekocht. Nach der Abkühlung wurde das erhaltene Gemisch abgenutscht, das Filtrat eingeengt, der Rückstand in Aether aufgenommen, 2 Mal mit Wasser gewaschen, über Na₂S0₄ getrocknet und anschliessend eingeengt. Auf diese Weise erhielt man das 2-Dimethylamino-4-äthylmerkaptomethyl-6-N,N-dimethylcarbamoyloxy-pyrimidin der Formel

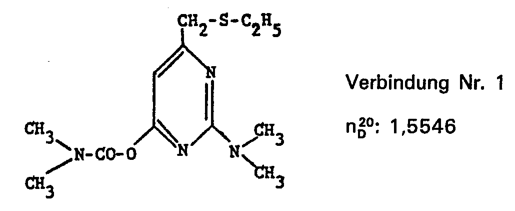

Analog zu den vorstehend beschriebenen Herstellungsverfahren können auch die folgenden Verbindungen der Formel I hergestellt werden:

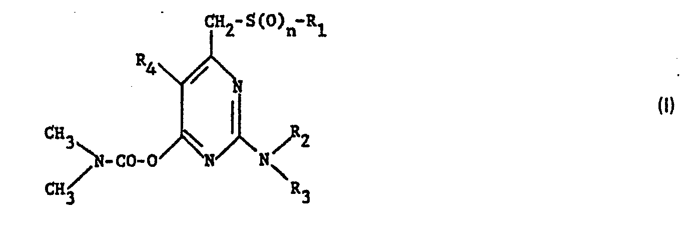

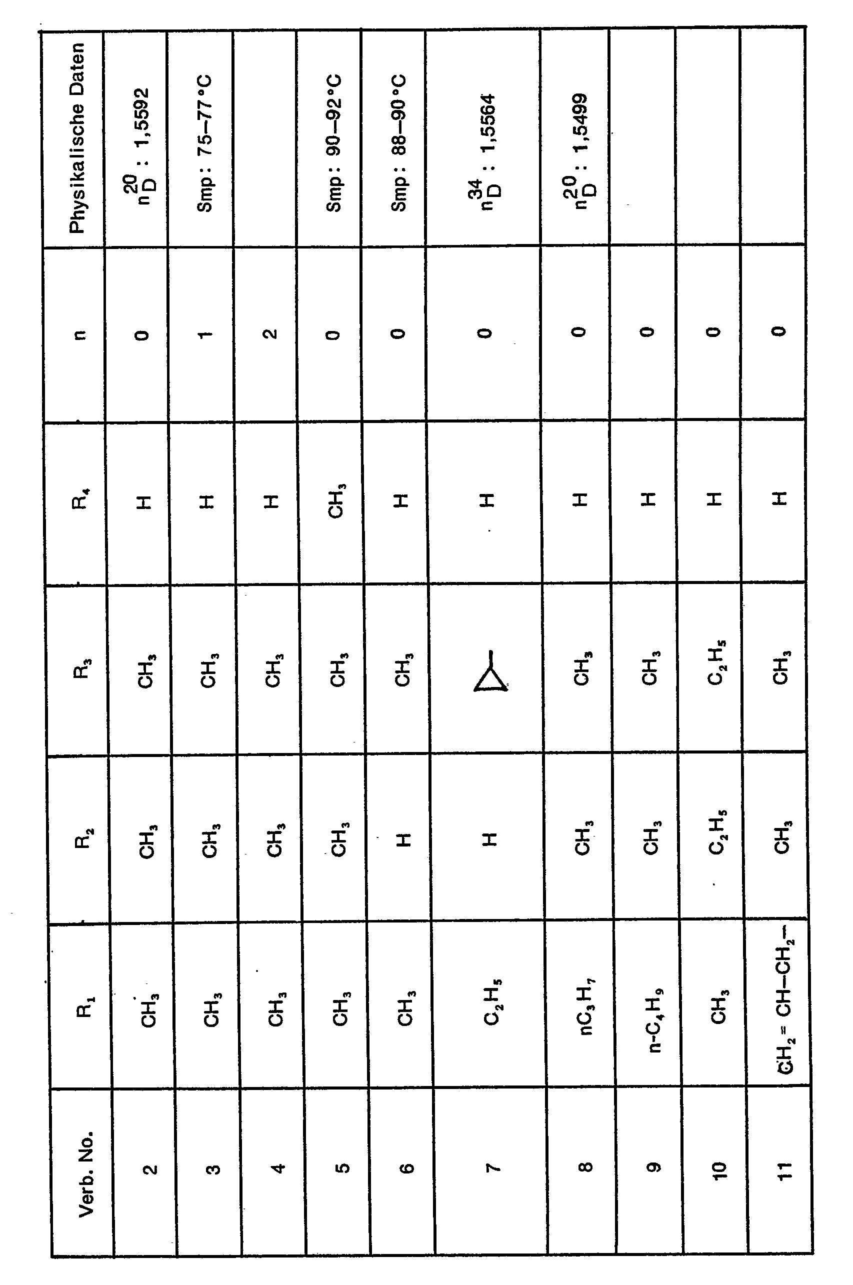

Beispiel 2: Insektizide Kontakt-Wirkung: Aphis craccivora und Myzus persicae.

In Töpfen angezogene Pflanzen (Vicia faba) wurden vor dem Versuchbeginn je mit ca. 200 Individuen der Spezies Aphis fabae oder Myzus persicae besiedelt. Die so behandelten Pflanzen wurden 24 Stunden später mit einer Lösung enthaltend 200 oder 100 ppm der zu prüfenden Verbindung bis zur Tropfnässe besprüht. Man verwendete pro Test-Verbindung und pro Konzentration zwei Pflanzen und eine Auswertung der erzielten Abtötungsrate erfolgte nach weiteren 24 Stunden.

Verbindungen gemäss Beispiel 1 zeigten im obigen Versuch eine positive Wirkung gegen Insekten der Spezies Aphis craccivora und Myzus persicae.

### Beispiel 3: Insektizide Wirkung (systemisch): Aphis craccivora

Bewurzelte Bohnenpflanzen wurden in Töpfe, welche 600 ccm Erde enthalten, verpflanzt und anschliessend 50 ml einer Lösung der zu prüfenden Verbindung (erhalten aus einem 25%igen Spritzpulver) in einer Konzentration von 50 ppm. oder 10 ppm. direkt auf die Erde aufgegossen.

Nach 24 Stunden wurden auf die oberirdischen Pflanzenteile Läuse der Spezies Aphis craccivora gesetzt und die Pflanzen mit einem Plastikzylinder überstülpt, um die Läuse vor einer eventuellen Kontakt- oder Gaswirkung der Testsubstanz zu schützen.

Die Auswertung der erzielten Abtötung erfolgte 48 und 72 Stunden nach Versuchsbeginn. Pro Konzentrationsdosis Testsubstanz wurden zwei Pflanzen, je eine in einem separaten Topf, verwendet. Der Versuch wurde bei 25°C und 70% relativer. Luftfeuchtigkeit durchgeführt.

Verbindungen gemäss Beispiel 1 zeigten im obigem Versuch eine gute systemische Wirkung gegen Insekten der Spezies Aphis craccivora.

## Patentansprüche (Patentansprüche für folgende(n) Vertragsstaat(en) : BE, CH, DE, FR, GB, IT, NL)

1. Eine Verbindung der Formel I 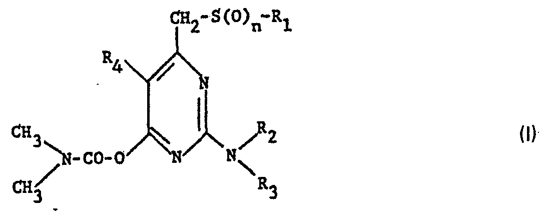 worin R, eine C₁-C₄-Alkyl- oder Allylgruppe, R₂ ein Wasserstoffatom oder eine Methyl- oder Aethylgruppe, R₃ eine Methyl-, Aethyl- oder Cyclopropylgruppe, R₄ ein Wasserstoffatom oder eine Methylgruppe und n die Zahl 0, 1 oder 2 bedeuten.

2. Verbindung nach Anspruch 1 der Formel I, worin R₁ eine C₁―C₃-Alkylgruppe bedeutet.

3. Verbindung nach Anspruch 2 der Formel worin R₁ eine Methyl- oder Aethylgruppe bedeutet.

4. Verbindung nach einem der Ansprüche 1 bis 3 der Formel I, worin n die Zahl 0 bedeutet.

5. Verbindung nach einem der Ansprüche 1 bis 4 der Formel worin R₂ ein Wasserstoffatom oder eine Methylgruppe, R3 eine Methylgruppe und R₄ ein Wasserstoffatom bedeuten.

6. Verbindung nach Anspruch 5 der Formel

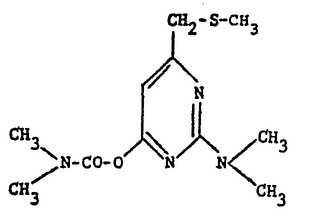

7. Verbindung nach Anspruch 5 der Formel

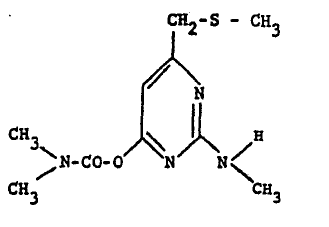

8. Verbindung nach Anspruch 5 der Formel

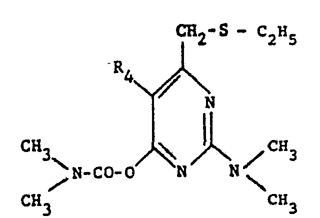

9. Verfahren zur Herstellung von in einem der Ansprüche 1 bis 8 genannten Verbindungen, dadurch gekennzeichnet, dass man eine Verbindung der Formel II 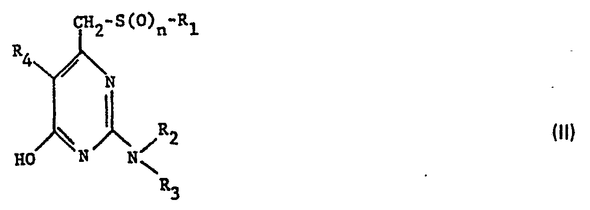 worin R_{1'} R₂, R_{3'} R₄ und n die entsprechenden, in einem der Ansprüche 1 bis 8 angegebenen Bedeutungen haben, in Gegenwart einer Base mit einem Dimethylcarbamoylhalid umsetzt oder eine Verbindung der Formel III 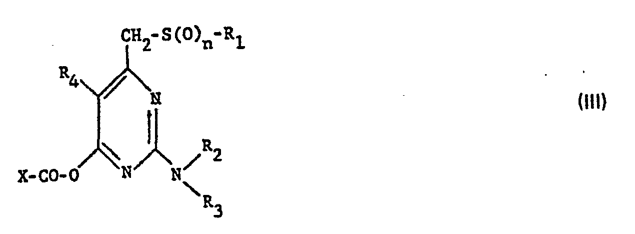 worin R_{1'} R₂, R₃, R₄ und n die oben angegebenen Bedeutungen haben und X ein Halogenatom darstellt, mit Dimethylamin umsetzt.

10. Insektizides Mittel enthaltend als aktive Komponente eine der in einem der Ansprüche 1 bis 8 genannten Verbindungen.

11. Verwendung von einer der in einem der Ansprüche 1 bis 8 genannten Verbindungen zur Bekämpfung von Insekten.

12. Verwendung nach Anspruch 11 zur Bekämpfung von Insekten der Familie Aphididae.

## Patentansprüche (Patentansprüche für folgende(n) Vertragsstaat(en) : AT)

1. Insektizides Mittel enthaltend als aktive Komponente eine Verbindung der Formel I - 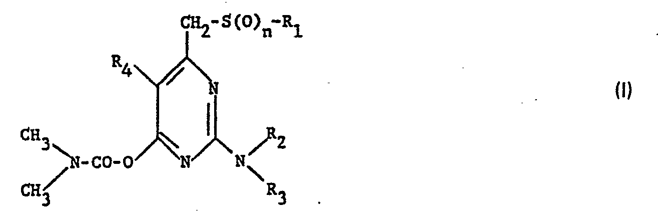 worin R₁ eine C₁―C₄-Alkyl- oder Allylgruppe, R₂ ein Wasserstoffatom oder eine Methyl- oder Aethylgruppe, R₃ eine Methyl-, Aethyl- oder Cyclopropylgruppe, R₄ ein Wasserstoffatom oder eine Methylgruppe und n die Zahl 0, 1 oder 2 bedeuten.

2. Insektizides Mittel nach Anspruch 1 enthaltend ais aktive Komponente eine Verbindung der Formel I, worin R₁ eine C₁―C₃-Alkylgruppe bedeutet.

3. Insektizides Mittel nach Anspruch 2 enthaltend als aktive Komponente eine Verbindung der Formel I, worin R₁ eine Methyl- oder Aethylgruppe bedeutet.

4. Insektizides Mittel nach einem der Ansprüche 1 bis 3 enthaltend als aktive Komponente eine Verbindung der Formel I, worin n die Zahl 0 bedeutet.

5. Insektizides Mittel nach einem der Ansprüche 1 bis 4 enthaltend als aktive Komponente eine Verbindung der Formel 1, worin R₂ ein Wasserstoffatom oder eine Methylgruppe, R₃ eine Methylgruppe und R₄ ein Wasserstoffatom bedeuten.

6. Insektizides Mittel nach Anspruch 5 enthaltend als aktive Komponente eine Verbindung der Formel

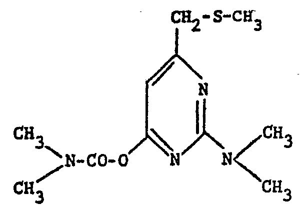

7. Insektizides Mittel nach Anspruch 5 enthaltend als aktive Komponente eine Verbindung der Formel

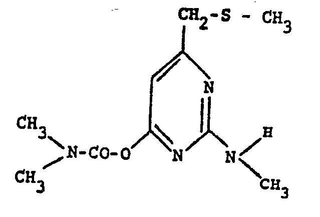

8. Insektizides Mittel nach Anspruch 5 enthaltend als aktive Komponente eine Verbindung der Formel

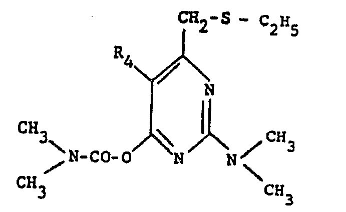

9. Verfahren zur Herstellung von in einem der Ansprüche 1 bis 8 definierten Verbindungen, dadurch gekennzeichnet, dass man eine Verbindung der Formel ll 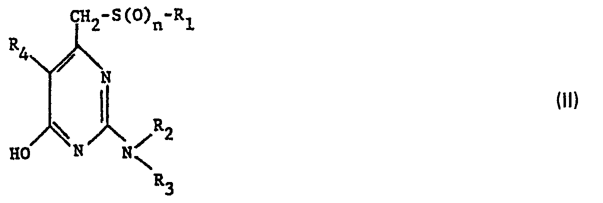 worin R₁, R_{2'} R_{3'} R₄ und n die entsprechenden, in einem der Ansprüche 1 bis 8 angegebenen Bedeutungen haben, in Gegenwart einer Base mit einem Dimethylcarbamoylhalid umsetzt oder eine Verbindung der Formel III 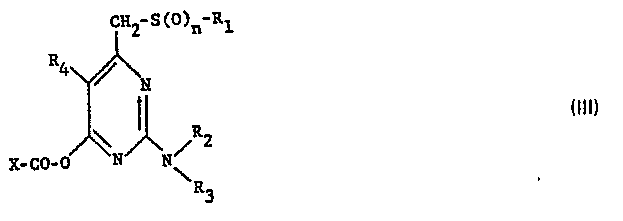 worin R₁, R₂, R₃, R₄ und n die oben angegebenen Bedeutungen haben und X ein Halogenatom darstellt, mit Dimethylamin umsetzt.

10. Verwendung von einer in einem der Ansprüche 1 bis 8 definierten Verbindungen zur Bekämpfung von Insekten.

11. Verwendung nach Anspruch 10 zur Bekämpfung von Insekten der Familie Aphididae.

## Claims (Claims for the following Contracting State(s) : BE, CH, DE, FR, GB, IT, NL)

1. A compound of the formula I 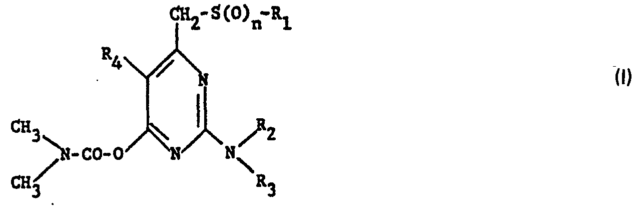 wherein R₁ is a C₁-C₄-alkyl or allyl group, R₂ is a hydrogen atom, or a methyl or ethyl group, R₃ is a methyl, ethyl or cyclopropyl group, R₄ is a hydrogen atom or a methyl group and n is zero or the integer 1 or 2.

2. A compound of the formula I as claimed in claim 1, wherein R₁ is a C₁―C₃-alkyl group.

3. A compound of the formula I as claimed in claim 2, wherein R₁ is a methyl or ethyl group.

4. A compound of the formula I as claimed in any one of claims 1 to 3, wherein n is zero.

5. A compound of the formula I as claimed in any one of claims 1 to 4 wherein R₂ is a hydrogen atom or a methyl group, R₃ is a methyl group and R₄ is a hydrogen atom.

6. A compound as claimed in claim 5 of the formula

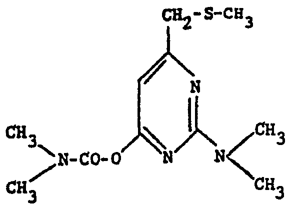

7. A compound as claimed in claim 5 of the formula

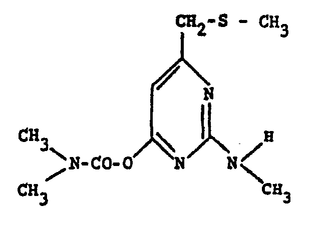

8. A compound as claimed in claim 5 of the formula

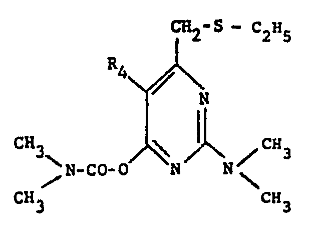

9. A process for producing a compound as claimed in any one of claims 1 to 8, characterised in that a compound of the formula II 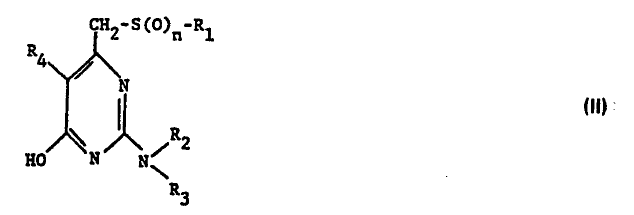 wherein R_{1'} R₂, R₃, R₄ and n have the corresponding meanings given in any one of claims 1 to 8, is reacted with a dimethylcarbamoyl halide in the presence of a base, or a compound of the formula III : 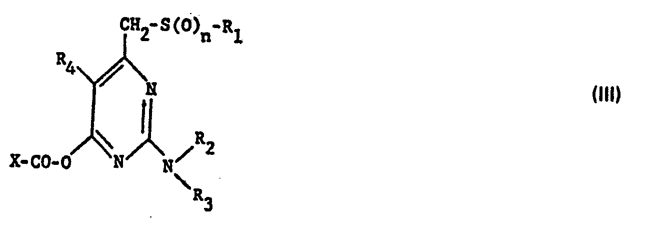 wherein R_{1'} R₂, R₃, R₄ and n have the meanings defined above, and X is a halogen atom, is reacted with dimethylamine.

10. An insecticidal composition containing as active ingredient a compound as claimed in any one of claims 1 to 8.

11. Use of a compound as defined in any one of claims 1 to 8 for combating insect pests.

12. Use according to claim 11 for combating insects of the family Aphididae.

## Revendications (Revendications pour l'(les) Etat(s) contractant(s) suivant(s) : BE, CH, DE, FR, GB, IT, NL)

1. Composé de formule l 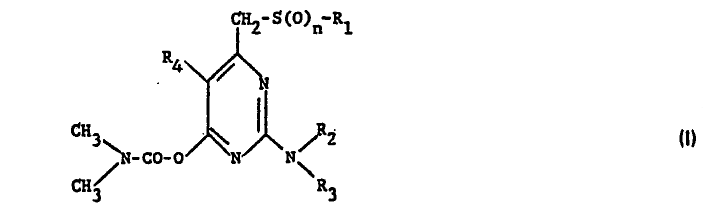 où R, représente un groupe alcoyle en C, à C₄ ou un groupe allyle, R₂ un atome d'hydrogène ou un groupe méthyle ou éthyle, R₃ un groupe méthyle, éthyle ou cyclopropyle, R₄ un atome d'hydrogène ou un groupe méthyle et n le nombre 0, 1 ou 2.

2. Composé selon la revendication 1 de formule I, où R₁ représente un groupe alcoyle en C₁ à C₃.

3. Composé selon la revendication 2 de la formule 1, où R₁, représente un groupe méthyle ou éthyle.

4. Composé selon l'une des revendications 1 à 3 de formule I, où n représente le nombre 0.

5. Composé selon l'une des revendications 1 à 4 de formule I, où R₂ représente un atome d'hydrogène ou un groupe méthyle, R₃ un groupe méthyle et R₄ un atome d'hydrogène.

6. Composé selon la revendication 5 de formule

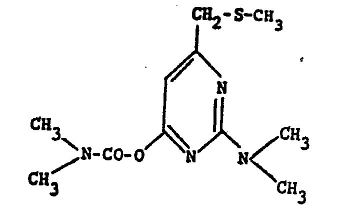

7. Composé selon la revendication 5 de formule

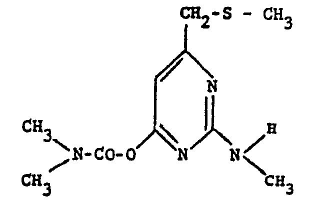

8. Composé selon la revendication 5 de formule

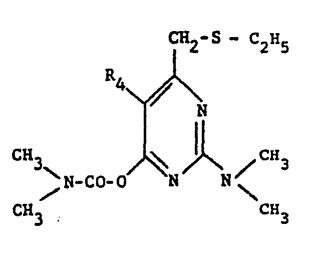

9. Procédé de préparation de composés mentionnés dans l'une des revendications 1 à 8, caractérisé en ce qu'on fait réagir un composé de formule Il 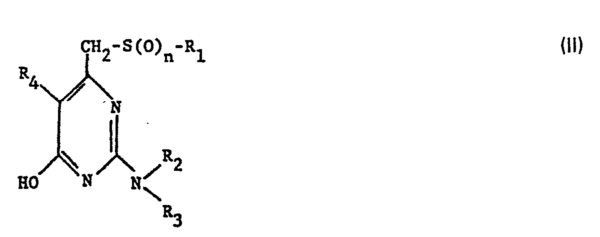 où R₁, R₂, R₃, R₄ et n ont les significations correspondantes, données dans l'une des revendications 1 à 8, en présence d'une base avec un halogénure de diméthylcarbamoyle ou en ce qu'on fait réagir un composé de formule III 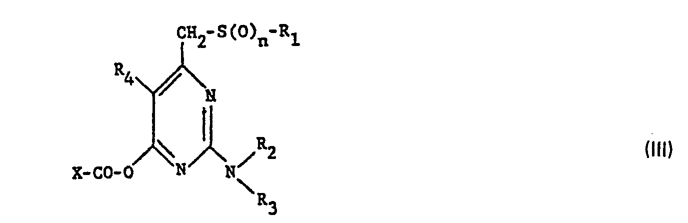 où R₁, R_{2'} R_{3'} R₄ et n ont les significations données ci-dessus et où X représente un atome d'halogène, avec de la diméthylamine.

10. Agent insecticide contenant comme composant actif l'un des composés mentionnés dans l'une des revendications 1 à 8.

11. Application de l'un des composés mentionnés dans l'une des revendications 1 à 8 à la lutte contre les insectes.

12. Application selon la revendication 11 à la lutte contre les insectes de la famille des Aphidiens.

## Claims (Claims for the following Contracting State(s) : AT)

1. An insecticidal composition containing as active ingredient a compound of the formula 1 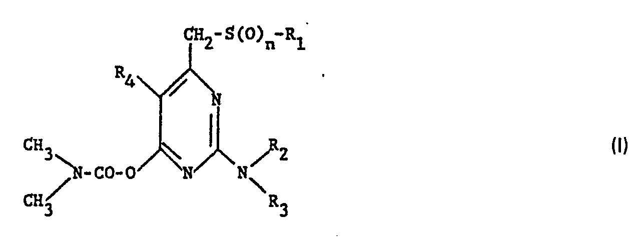 wherein R₁ is a C₁―C₄-alkyl or allyl group, R₂ is a hydrogen atom or a methyl or ethyl group, R₃ is a methyl, ethyl or cyclopropyl group, R₄ is a hydrogen atom or a methyl group, and n is zero or the integer 1 or 2.

2. An insecticidal composition as claimed in claim 1, containing as active ingredient a compound of the formula 1 wherein R₁ is a C₁―C₃ alkyl group.

3. An insecticidal composition as claimed in claim 2, containing as active ingredient a compound of the formula 1 wherein R, is a methyl or ethyl group.

4. An insecticidal composition as claimed in any one of claims 1 to 3, containing as active ingredient a compound of the formula 1 wherein n is zero.

5. An insecticidal composition as claimed in any one of claims 1 to 4, containing as active ingredient a compound of the formula l wherein R₂ is a hydrogen atom or a methyl group, R₃ is a methyl group, and R₄ is a hydrogen atom.

6. An insecticidal composition as claimed in claim 5, containing as active ingredient a compound of the formula

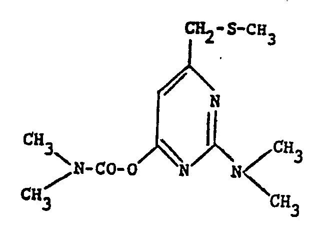

7. An insecticidal composition as claimed in claim 5, containing as active ingredient a compound of the formula

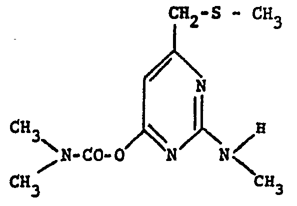

8. An insecticidal composition as claimed in claim 5, containing as active ingredient a compound of the formula

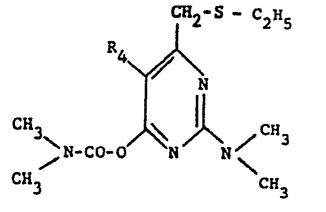

9. A process for producing a compound as claimed in any one of claims 1 to 8, characterised in that a compound of the formula Il 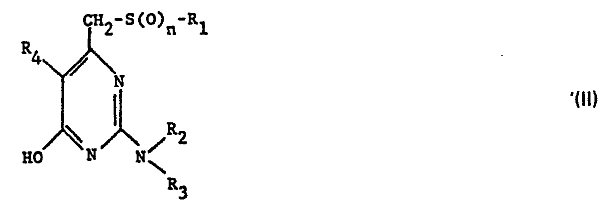 wherein R₁, R₂, R₃, R₄ and n have the corresponding meanings given in any one of claims 1 to 8, is reacted with a dimethylcarbamoyl halide in the presence of a base, or a compound of the formula III 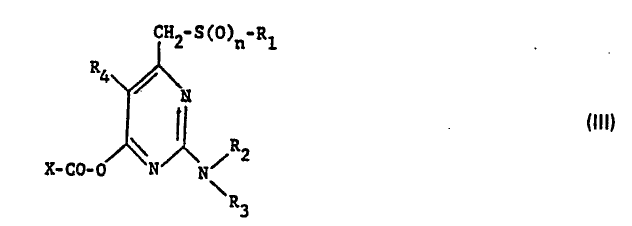 wherein R₁, R₂, R₃, R₄ and n have the meanings defined above, and X is a halogen atom, is reacted with dimethylamine.

10. Use of a compound as defined in any one of claims 1 to 8 for combating insect pests.

11. Use according to Claim 10 for combating insects of the family Aphididae.

## Revendications (Revendications pour l'(les) Etat(s) contractant(s) suivant(s) : AT)

1. Agent insecticide contenant comme composant actif un composé de formule 1 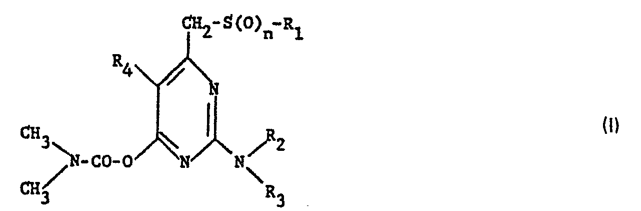 où R₁ représente un groupe alcoyle en C₁ à C₄ ou un groupe allyle, R₂ un atome d'hydrogène ou un groupe méthyle ou éthyle, R₃ un groupe méthyle, éthyle ou cyclopropyle, R₄ un atome d'hydrogène ou un groupe méthyle et n le nombre 0, 1 ou 2.

2. Agent insecticide selon la revendication 1 contenant comme composant actif un composé de formule I, où R₁ représente un groupe alcoyle en C₁ à C₃.

3. Agent insecticide selon la revendication 2 contenant comme composant actif un composé de formule I, où R₁ représente un groupe méthyle ou éthyle.

4. Agent insecticide selon l'une des revendications 1 à 3 contenant comme composant actif un composé de formule I, où n représente le nombre 0.

5. Agent insecticide selon l'une des revendications 1 à 4 contenant comme composant actif un composé de formule I, où R₂ représente un atome d'hydrogène ou un groupe méthyle, R₃ un groupe méthyle et R₄ un atome d'hydrogène.

6. Agent insecticide selon la revendication 5 contenant comme composant actif un composé de formule

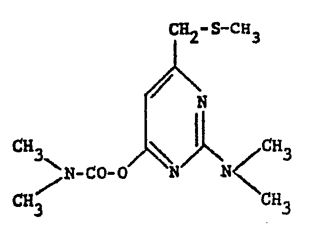

7. Agent insecticide selon la revendication 5 contenant comme composant actif un composé de formule

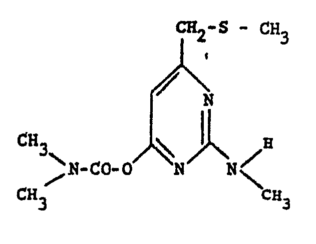

8. Agent insecticide selon la revendication 5 contenant comme composant actif un composé de formule

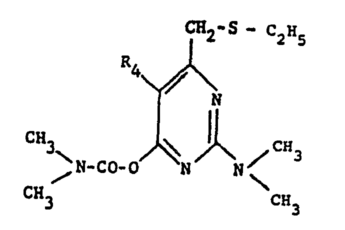

9. Procédé de préparation de composés définis dans l'une des revendications 1 à 8, caractérisé en ce qu'on fait réagir un composé de formule ll 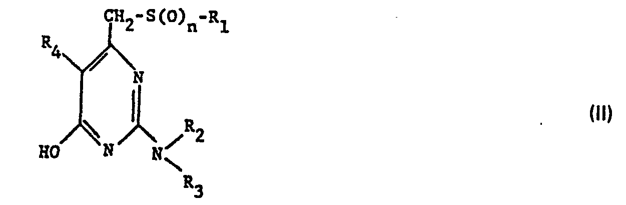 où R₁, R₂, R₃, R₄ et n ont les significations correspondantes, données dans l'une des revendications 1 à 8, en présence d'une base avec un halogénure de diméthylcarbamoyle ou un composé de formule III 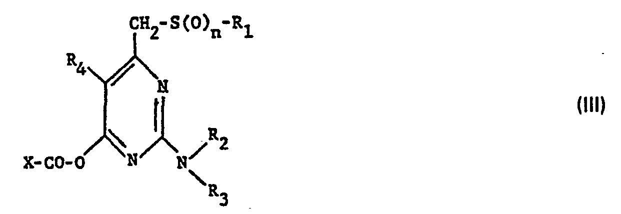 où R₁, R₂, R₃, R₄ et n ont les significations données ci-dessus et où X représente un atome d'halogène, avec de la diméthylamine.

10. Application de l'un des composés définis dans l'une des revendications 1 à 8 à la lutte contre les insectes.

11. Application selon la revendication 10 à la lutte contre les insectes de la famille des Aphidiens.
